# DEMANDE DE BREVET EUROPEEN

(11) **EP 3 970 761 A1**
(43) Date de publication de la demande: **23.03.2022**
(21) Numéro de dépôt: 20315416.6
(22) Date de dépôt: 22.09.2020
(51) Int. Cl.: A61L 27/34, A61L 27/54, A61L 27/56

(54) **DISPOSITIF MÉDICAL IMPLANTABLE POUR LA RÉPARATION OSSEUSE**

(71) Demandeur: Institut Polytechnique de Grenoble, 38000 Grenoble (FR); Centre national de la recherche scientifique, 75016 Paris (FR); Centre Hospitalier Universitaire Grenoble Alpes, 38700 La Tronche (FR); Centre Hospitalier Annecy Genevois, 74370 Epagny Metz-Tessy (FR)
(72) Inventeur: Picart, Catherine, 38000 Grenoble (FR); Bettega, Georges, 38000 Grenoble (FR); Bouyer, Michael, 74320 Sevrier (FR)
(74) Mandataire: Brizio Delaporte, Allison

(57) **Abrégé**

L'invention concerne un dispositif médical implantable pour la réparation osseuse d'une perte de substance osseuse comprenant :
- un échafaudage présentant une structure tridimensionnelle et comprenant au moins un polymère,
- un film comprenant au moins une protéine de la famille des Bone Morphogenetic Proteins (BMPs),
caractérisé en ce que l'échafaudage définit un volume intérieur comprenant un maillage tridimensionnel délimitant des pores, les pores étant ouverts et interconnectés, chaque pore ayant une plus grande dimension supérieure à 200µm, l'échafaudage présentant une porosité minimale de 80%, et en ce que le film revêt le maillage tridimensionnel.

La présente invention concerne le domaine des dispositifs médicaux implantables. Elle trouve pour application particulièrement avantageuse le domaine de la réparation osseuse suite à une perte de substance osseuse. L'invention s'applique particulièrement aux défauts osseux de grand volume de 2 cm³ à 15 cm³.

## Description

### DOMAINE TECHNIQUE DE L'INVENTION

La présente invention concerne le domaine des dispositifs médicaux implantables. Elle trouve pour application particulièrement avantageuse le domaine de la réparation osseuse suite à une perte de substance osseuse. L'invention s'applique particulièrement aux défauts osseux de grand volume de 2 cm³ à 15 cm³.

### ÉTAT DE LA TECHNIQUE

À ce jour, les greffes osseuses autologues restent la principale solution clinique pour traiter les pertes osseuses étendues et les traumatismes, mais elles sont entravées par plusieurs inconvénients, notamment une disponibilité limitée, des douleurs pour le patient, un temps de cicatrisation supplémentaire et une morbidité du site donneur.

L'ingénierie tissulaire à l'aide d'échafaudages synthétiques, de facteurs bioactifs et/ou de cellules souches offre des stratégies thérapeutiques alternatives et est prometteuse pour la régénération osseuse.

Ces techniques ne sont toutefois toujours pas adaptées à la réparation des gros défauts osseux (environ 5 cm³) qui reste difficile. En particulier, pour les gros défauts osseux, un échafaudage synthétique structurel peut ne pas être suffisant pour permettre une régénération complète.

Les céramiques, notamment les composites d'hydroxyapatite (HAP) et de phosphate tricalcique (TCP) sont les échafaudages les plus biomimétiques, mais sont fragiles et présentent une biodégradabilité variable. De plus, ils induisent un niveau basique de formation osseuse. Les métaux tels que le titane sont intéressants pour leurs propriétés mécaniques, mais leur grande élasticité, supérieure à celle de l'os, génère des contraintes et ils ne sont pas biodégradables.

L'utilisation de polymères s'est développée, compte tenu de leur polyvalence, de leurs propriétés mécaniques modulables et de leur biodégradabilité. À ce jour, le polycaprolactone (PCL) et les dérivés d'acide poly(lactique) (PLA) sont les plus largement utilisés dans l'ingénierie des tissus osseux.

Très intéressant, le développement récent de la fabrication additive permet de concevoir des échafaudages architecturés 3D sur mesure, adaptables à la taille du défaut et plus faciles à mettre en œuvre dans une perspective réglementaire. Les polymères sont particulièrement bien adaptés à la fabrication additive d'échafaudages. Ils peuvent être fabriqués sous forme de filaments et être imprimés en 3D en utilisant plusieurs techniques, notamment l'impression par dépôt de fil fondu modélisation par dépôt fondu (FDM). L'échafaudage architecturé en 3D joue le rôle d'un remplisseur d'espace qui devrait être mécaniquement stable pour permettre la croissance osseuse à l'intérieur des pores de l'échafaudage.

Cependant, pour les grandes zones de défauts, un échafaudage structurel peut ne pas être suffisant pour permettre une régénération complète.

Dans ces cas, des cellules souches ou des facteurs exogènes peuvent être ajoutés à l'échafaudage afin d'améliorer la régénération. L'utilisation de cellules souches en combinaison avec des échafaudages semble avoir un potentiel compte tenu de leur sécrétion de facteurs, mais est plus compliquée à mettre en place, car différentes étapes sont nécessaires pour récolter les cellules des patients, les étendre en culture et enfin, les réimplanter dans le patient.

Comme alternative à l'implantation de cellules souches, l'utilisation de facteurs de croissance vise à recruter des cellules souches directement sur le site d'implantation. Jusqu'à présent, BMP-2 a été la protéine approuvée cliniquement la plus étudiée en raison de sa capacité à cibler directement les récepteurs BMP à la surface cellulaire et à déclencher la différenciation des cellules souches en cellules osseuses.

Une étude précédente, Bouyer M, Guillot R, Lavaud J, Plettinx C, Olivier C, Curry V, et al. Surface delivery of tunable doses of BMP-2 from an adaptable polymeric scaffold induces volumetric bone regeneration. Biomaterials. 104 (2016) 168-81 a montré qu'il est possible de réparer un défaut osseux fémoral de taille critique chez le rat inférieur à 2 cm³ en combinant un tube polymérique creux à un revêtement de surface ostéoinductif en utilisant un film polyélectrolyte comme support BMP-2.

Un objet de la présente invention est donc de proposer un implant médical implantable optimisé pour la réparation volumique de grande taille.

Les autres objets, caractéristiques et avantages de la présente invention apparaîtront à l'examen de la description suivante et des dessins d'accompagnement. Il est entendu que d'autres avantages peuvent être incorporés.

### RÉSUMÉ DE L'INVENTION

Pour atteindre cet objectif, selon un mode de réalisation on propose un dispositif médical implantable pour la réparation osseuse d'une perte de substance osseuse comprenant : un échafaudage présentant une structure tridimensionnelle et comprenant avantageusement au moins un polymère, un film comprenant au moins une protéine de la famille des Bone Morphogenetic Proteins(BMPs), caractérisé en ce que l'échafaudage définit un volume intérieur comprenant un maillage tridimensionnel délimitant des pores, avantageusement les pores étant ouverts et interconnectés, avantageusement chaque pore ayant une plus grande dimension supérieure à 200µm, préférentiellement de l'ordre de 2000µm, l'échafaudage présente une porosité minimale de 80%, et que le film revêt le maillage tridimensionnel.

Cette disposition assure une repousse homogène de l'os au sein de l'échafaudage. Ledit échafaudage assure une circulation des fluides notamment le sang qui va arriver au sein de l'échafaudage lors de son implantation au sein du défaut osseux. La porosité de l'échafaudage assure qu'il puisse être recouvert de façon avantageusement homogène par le film. Le film épouse avantageusement parfaitement le maillage tridimensionnel de l'échafaudage. Le maillage tridimensionnel est pelliculé par le film.

L'échafaudage est également avantageusement suffisamment résistant mécaniquement pour tenir seul au sein du défaut volumique osseux. De plus son architecture en 3 dimensions lui permet de résister aux contraintes mécaniques, notamment à la compression.

La porosité de l'échafaudage est de préférence telle que l'échafaudage puisse être recouvert de façon homogène par le film. Avantageusement, le film est déposé au moyen d'un procédé automatisé. Ainsi, le film épouse parfaitement la surface de la structure tridimensionnelle de l'échafaudage.

Cette coopération optimisée de l'échafaudage grâce à son architecture 3 dimensions et sa porosité avec le film assure une réparation osseuse efficace des défauts volumiques osseux et notamment ceux de taille critique et de gros volume par exemple de taille comprise entre 2 cm³ et 15 cm³.

L'utilisation du film comme revêtement permet de découpler l'architecture de l'échafaudage 3D du film qui est ostéoinductieur.

L'invention permet de contrôler indépendamment la porosité de l'échafaudage et le chargement de facteurs ostéoinducteurs de sorte à moduler le relargage desdits facteurs permettant in fine d'assurer une croissance osseuse homogène et de qualité.

### BRÈVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La figure 1 représente un échafaudage en PLA imprimé en 3D selon l'invention.
La figure 2A représente une imagerie macroloupe par contraste de phase d'un dispositif selon l'invention comprenant un échafaudage PLA imprimé en 3D et recouvert d'un film selon l'invention.
La figure 2B représente une image confocale par contraste de phase d'une section d'un dispositif selon la figure 2A pour visualiser les filaments pelliculés de l'échafaudage.
La figure 2C représente une image par microscopie électronique à balayage (MEB) des entretoises de l'échafaudage.
La figure 2D représente une image par microscopie électronique à balayage (MEB) d'une rayure réalisée à l'aide d'une aiguille pour évaluer la présence du film.
La figure 2E représente la quantification en utilisant un test µBCA du chargement de BMP-2 dans un film (PLL / HA) de 24 couches de chargement en fonction de la concentration initiale de BMP-2 dans la solution de chargement.
La figure 2F représente la quantification de la libération de BMP-2 en fonction de la concentration de BMP-2 initialement chargée, pour deux niveaux de réticulation EDC30 et EDC70.

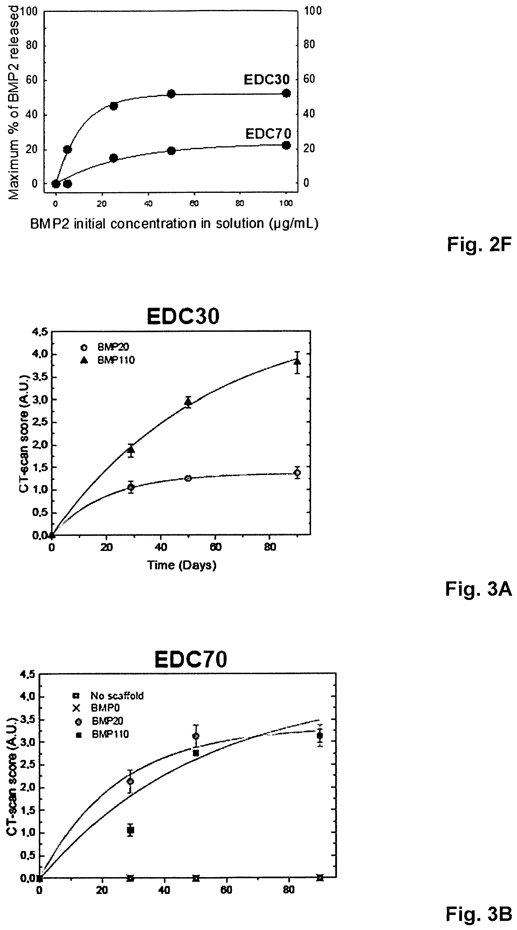

Les figures 3A à 3C représentent la quantification cinétique de la formation osseuse à l'aide de coupes scanner X ou de coupes tomodensitométriques obtenues avec deux niveaux de réticulation et deux doses de BMP-2. Les films EDC 30 et ED70 chargés avec des doses de BMP-2 (20 et 110 µg/cm³ d'échafaudage) ont été comparés. Deux contrôles négatifs ont été ajoutés : implant pelliculé sans BMP et perte de substance sans complément. CT-scores calculés à partir des coupes tomodensitométriques en fonction du temps et des ajustements exponentiels correspondants aux données pour les groupes EDC30 (Figure 3A) et EDC70 (figure 3B). La valeur de plateau (Bmax), le temps caractéristique (T) déduits des ajustements et la qualité d'ajustement R² sont donnés dans les tableaux correspondants Table 1 et Table 2.
La figure 3C représente le volume osseux total en fonction du temps (D29, D50 et D90) en fonction de la dose de BMP-2, pour les deux films réticulés EDC30 et EDC70. Les ajustements linéaires sont également représentés.
La figure 4 représente des reconstructions 3D à partir des coupes tomodensitométriques montrant la cinétique de régénération osseuse pour quatre conditions représentatives : contrôle négatif (Ctrl -) (échafaudage pelliculé sans BMP-2 dans le film), échafaudage pelliculé à faible dose de BMP-2 (BMP50, LD) et une dose élevée de BMP-2 (BMP110, HD) et une greffe osseuse (BG). La longueur totale de l'implant, correspondant à la distance du bord inférieur de la mandibule au niveau de la perte de substance osseuse, est de 4 cm, direction X sur le schéma.

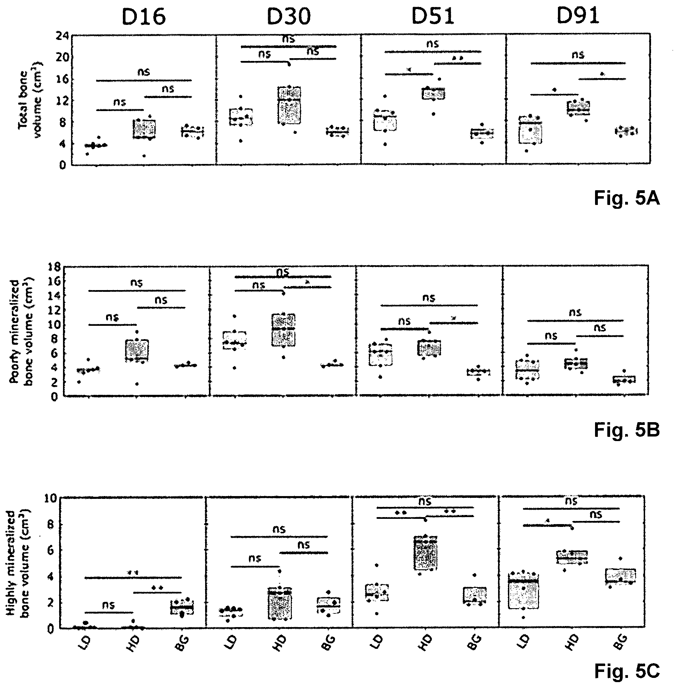

Les figures 5A à 5E illustrent l'analyse quantitative de la cinétique de formation osseuse suivie sur les coupes tomodensitométriques pour les films EDC30 chargés avec deux doses de BMP-2. Les échafaudages pelliculés ont été chargés de BMP-2 à 50 (LD, n = 6) et 110 µg/cm³ (HD, n = 5) et leur capacité de régénération osseuse a été comparée à l'autogreffe osseuse BG, (n = 4). Les figures 5A à 5C représentent les données en box plot du volume osseux total (figure 5A), des volumes osseux peu minéralisés (figure 5B) et hautement minéralisés (figure 5C) en fonction de la dose de BMP-2 LD par rapport à HD en comparaison à BG.
La figure 5D représente des scores tomodensitométriques en fonction du temps et des ajustements exponentiels correspondants aux données pour les films EDC30 ; la valeur de plateau correspondante (Bmax), le temps caractéristique (T) déduit des ajustements et la qualité d'ajustement R² sont donnés dans la TABLE 3.
La figure 5E représente le pourcentage d'os en dehors de l'implant (appelé «os ectopique») en fonction du temps pour LD et HD. * p <0,05; ** p <0,01.
Les figures 6A à 6E illustrent l'analyse quantitative par micro tomodensitométrie de la formation osseuse prise à 3 mois (J91), après explantation. La figure 6A représente le contrôle négatif (ctrl -échafaudage pelliculé EDC30 sans BMP-2), les échafaudages pelliculés contenant des BMP-2 à faible dose LD et haute dose HD, et greffe osseuse BG.
La figure 6B illustre une représentation en boite à moustache (ou box plot en anglais) du volume osseux total.
La figure 6C est une représentation en boite à moustache (ou box plot en anglais) en fonction de la dose totale de BMP-2 par implant.
La figure 6D est une représentation en boite à moustache (ou box plot en anglais) de la densité minéralisée osseuse (DMO).
La figure 6E est une représentation en boite à moustache (ou box plot en anglais) du score d'homogénéité mesuré pour la LD et la HD.
Dans chaque case des figures 6B à 6E, le coefficient de variation des données est donné en %. * p <0,05; ** p <0,01.
La figure 7A illustre une analyse quantitative de la surface osseuse sur la surface totale (BA / TA). À la surface de l'os à l'intérieur de chaque section de l'implant (S1, S2, S3).
La figure 7B illustre le pourcentage de l'os à l'intérieur de chaque section de l'implant (S1, S2, S3).

Les dessins sont donnés à titre d'exemples et ne sont pas limitatifs de l'invention. Ils constituent des représentations destinées à faciliter la compréhension de l'invention.

### DESCRIPTION DÉTAILLÉE DE L'INVENTION

Avant d'entamer une revue détaillée de modes de réalisation de l'invention, sont énoncées ci-après des caractéristiques optionnelles qui peuvent éventuellement être utilisées en association ou alternativement :
Selon un exemple, l'échafaudage est inerte. Il ne possède pas de capacité à induire la repousse osseuse c'est-à-dire qu'il n'est pas ostéoinducteur. L'échafaudage joue un rôle de support du film et de support passif pour la repousse osseuse. L'échafaudage a un rôle ostéo conducteur en guidant la repousse osseuse.

Selon un exemple, l'échafaudage comprend au moins un polymère choisi parmi le groupe constitué de l'acide polylactique (PLA) ou la polycaprolactone (PCL) ou l'acide Poly(glycolique) (PGA) ou des copolymères ou des mélanges de ceux-ci.

Selon un exemple, le maillage tridimensionnel présente des angles d'orientation compris entre 0°et 120°.

Selon un exemple, le film comprend une quantité de protéine BMPs comprise entre 0,017mg/cm³ à 0,072mg/cm³.

Cette sélection de la dose de BMP est particulièrement intéressante, car elle très nettement inférieure aux quantités de l'état de la technique ce qui est surprenant par rapport à l'idée qu'une plus forte dose serait bénéfique pour la réparation osseuse. La dose sélectionnée de BMP a permis de manière surprenante d'éviter d'éventuels effets secondaires comme une inflammation autour de l'implant et la production d'os en dehors de l'implant (os ectopique).

Selon un exemple, le film est un film polyélectrolyte, plus précisément le film comprend des polyélectrolytes.

Selon un exemple, le film est un film polyélectrolyte multicouche réticulé.

Selon un exemple, le film est réticulé par un agent de réticulation, préférentiellement 1-Éthyl-3-(3-diméthylaminopropyl) carbodiimide hydrochloride (EDC), préférentiellement à une concentration comprise entre 30 mg/mL (EDC30) et 70mg/ml (EDC70).

Selon un exemple, le film réticulé à une concentration comprise entre 30 mg/mL et 70mg/mL d'1-Éthyl-3-(3-diméthylaminopropyl)carbodiimide hydrochloride (EDC).

Selon un exemple, le dispositif est adapté pour la réparation osseuse de volume compris entre 2 cm³ et 15 cm³. Préférentiellement, supérieur à 6 cm³, plus préférentiellement supérieur à 10 cm³.

Selon un aspect, l'invention concerne un procédé de fabrication d'un dispositif médical implantable tel que décrit ci-dessus caractérisé en ce que l'échafaudage est fabriqué par impression 3D.

Selon un exemple, le procédé de fabrication e comprend une étape de stérilisation du dispositif médical implantable.

Le dispositif médical implantable selon l'invention est destiné à la réparation osseuse.

Le dispositif médical selon l'invention est destiné à être implanté dans le corps humain ou animal, plus précisément dans un défaut osseux. En particulier, le dispositif est particulièrement efficace lorsque le défaut osseux est un défaut volumique. Selon une possibilité, le défaut est de taille critique. On entend par critique que le défaut osseux ne peut pas se combler spontanément par les seuls mécanismes habituels de cicatrisation. Avantageusement, le dispositif selon l'invention est particulièrement adapté au gros défaut osseux. On entend par gros défaut osseux que le défaut volumique osseux est supérieur à 2 cm³, plus précisément supérieure à 6 cm³, plus préférentiellement supérieure à 10 cm³ et avantageusement jusqu'à 15 cm³.

Le dispositif implantable selon l'invention comprend un échafaudage et un film recouvrant ledit échafaudage et comprenant avantageusement une protéine de la famille des protéines osseuses morphogénétique ou Bone Morphogenetic Proteins en anglais(BMP). Le dispositif médical implantable selon l'invention est également dénommé dispositif activé ou implant actif lorsqu'il comprend des protéines BMP.

L'échafaudage présente une structure tridimensionnelle avantageusement destiné à être inséré dans le défaut osseux à réparer. L'échafaudage est destiné à assurer un rôle de comblement au moins partiel du défaut osseux et préférentiellement total.

On entend par structure tridimensionnelle que l'échafaudage présente une organisation tridimensionnelle encore appelée conformation en 3 dimensions. La conformation en 3 dimensions va au-delà de la définition du pourtour de l'échafaudage et définit également la structure du volume intérieur de l'échafaudage.

L'échafaudage est avantageusement de forme complémentaire au défaut osseux à réparer. L'échafaudage assure également le support du film et sert de guide à la repousse osseuse.

L'échafaudage est un solide. C'est une forme géométrique possédant s 3 dimensions : hauteur (Y), largeur (Z), profondeur (X). Selon une possibilité, le rapport de forme, c'est à dire l'une des dimensions Pa rapport à l'autre est inférieur à 100.

À titre d'exemple, l'échafaudage est un pavé par exemple de profondeur (X) de 4 cm, de largeur (Z) de 3 cm, d'épaisseur (Y) de 1 cm soit un volume de 12 cm³.

L'échafaudage définit un volume intérieur. L'échafaudage comprend un maillage délimitant des pores. Avantageusement, le maillage s'étend dans l'ensemble du volume de l'échafaudage. Le maillage s'étend au sein de l'échafaudage dans le volume intérieur. Le maillage est tridimensionnel. Il s'étend dans le volume intérieur suivant les trois dimensions de l'échafaudage.

Avantageusement, l'échafaudage est dit poreux. Préférentiellement, l'échafaudage présente une porosité de minimum 80 %. Préférentiellement de l'ordre de 85 %, plus préférentiellement de l'ordre de 90 %. Cette porosité est notamment due au maillage.

La porosité de l'échafaudage s'entend comme le volume vide du volume intérieur de l'échafaudage.

Cette grande porosité de l'échafaudage assure une colonisation optimale de l'échafaudage par exemple par des cellules progénitrices puis par l'os au cours du temps tout en assurant un rôle de support mécanique.

L'échafaudage comprend des pores qui sont avantageusement ouverts c'est-à-dire que les pores sont reliés entre eux. Dans ce cas-là, les pores forment des canaux. Les pores sont alors également dénommés comme étant interconnectés. Ce type de porosité permet la circulation des fluides au sein de l'échafaudage.

Préférentiellement, les pores de l'échafaudage présentent au moins une dimension supérieure à 200µm, préférentiellement de l'ordre de 2000µm, à plus ou moins 10%. À titre préféré, la dimension est la plus grande dimension du pore. Selon une possibilité, la plus grande dimension du pore s'entend dans un plan parallèle au plan dans lequel s'étendent les filaments ou trabecules formant le maillage décrit ci-après.

L'échafaudage selon l'invention permet de fournir un support mécanique temporaire à la réparation osseuse, le temps que l'os se forme et croisse au sein des pores.

Préférentiellement, l'échafaudage est une structure inerte, c'est-à-dire que l'échafaudage seul n'assure pas d'ostéoinduction.

Selon une possibilité, l'échafaudage est biodégradable. Selon une possibilité, l'échafaudage est biorésorbable.

L'échafaudage comprend avantageusement au moins un matériau inerte, préférentiellement l'échafaudage est constitué de matériaux inertes. À titre d'exemple préféré, l'échafaudage comprend au moins un polymère choisi parmi l'acide polylactique (PLA) ou la polycaprolactone (PCL) ou l'acide polyglycolique (PGA), ou des copolymères ou des mélanges de ceux-là. Ils possèdent également des propriétés mécaniques intéressantes. L'échafaudage est dénommé échafaudage polymérique.

Préférentiellement, l'échafaudage est architecturé en 3 dimensions par exemple par une fabrication par impression en 3D notamment utilisant un dépôt de fil fondu ou Fused déposition modeling en anglais (FDM). Les échafaudages imprimés en 3D sont particulièrement adaptés à la réparation de gros défauts osseux. L'échafaudage peut s'adapter à la géométrie même complexe du défaut osseux à combler. On entend que l'échafaudage est architecturé c'est-à-dire qu'il est fabriqué ou construit ou agencé comme un tout organisé de sorte à lui donner un caractère architectural défini.

À titre d'exemple, le maillage de l'échafaudage est formé par des trabécules ou filaments. Les filaments ou trabécules sont disposés suivant différentes orientations permettant notamment de contrôler la porosité. L'orientation peut être de 0°, 90°, 15°, 30°, 45°, 60° ou bien- 15°/+15°, -30°/+30°, -45°/+45°, -60°/+60°, 60°/120°. La disposition des filaments ou trabecules se croissant définit des entretoises.

Selon une possibilité, l'échafaudage comprend des filaments. Les filaments forment le maillage de l'échafaudage. À titre d'exemple, les filaments sont en matériau inerte tel que le PLA présentant avantageusement un diamètre de 400 µm. Préférentiellement, les filaments sont espacés définissant ainsi un espacement des filaments, c'est-à-dire une entretoise comprise entre 1 et 2,5 mm.

L'échafaudage est particulièrement adapté à être recouvert par un film, préférentiellement un film polyélectrolyte, préférentiellement un film multicouches de polyélectrolytes.

Les films multicouches polyélectrolytes sont formés par la réticulation par réaction de condensation de groupes complémentaires situés sur la couche adjacente. Le document EP 1 535 952 décrit un procédé pratique pour produire des films multicouches polyélectrolytes réticulés.

Le document EP 1 535 952 décrit un film multicouche polyélectrolyte réticulé, dont le procédé de fabrication comprenant la réaction de paires de couches d'un polymère anionique avec des groupes carboxyliques et d'un polymère cationique avec des groupes amino en présence d'un agent de couplage carbodiimide.

Le film polyélectrolyte est plus préférablement biocompatible. En particulier, de tels films biocompatibles peuvent rendre toute surface revêtue biocompatible. Par conséquent, de tels matériaux biocompatibles lorsqu'ils sont appliqués sur des tissus biologiques, en particulier à l'intérieur du corps, présentent l'avantage de ne pas irriter les tissus environnants, de ne pas provoquer de réponse inflammatoire anormale et de ne pas provoquer de réaction allergique ou immunologique.

Le film polyélectrolyte comprend avantageusement deux couches ou plus, le film est donc dénommé film multicouches de polyélectrolytes.

Préférentiellement, chaque couche supplémentaire ayant la charge opposée de la couche précédente. L'architecture du film est conçue avec précision et peut être contrôlée avec une précision de 1 nm avec une plage de 1 à 50 000 nm, de préférence de 100 nm à 30 µm et avec une connaissance précise de sa composition moléculaire.

Le nombre de paires de couches dans un film multicouche de polyélectrolyte préparé peut varier dans une large plage et dépend de l'épaisseur souhaitée. En particulier, le nombre de paires de couches peut varier de 5 à 2000, de préférence de 5 à 1000, plus préférablement de 5 à 100, préférentiellement de 20 à 30, préférentiellement 24.

Comme indiqué ci-dessus, l'épaisseur du film peut généralement varier de 1 nm à 50 000 nm. Un film est considéré comme un film épais lorsque son épaisseur est supérieure à 300 nm. Selon l'invention et dans un mode de réalisation particulier, l'épaisseur du film est de 500 nm à 20 µm, plus préférentiellement de 1 à 10 µm.

Dans un aspect particulier de l'invention, le polymère cationique comprenant un groupe amino est la poly (L-lysine) (ou PLL) ou le polymère anionique comprenant un groupe amino est l'acide hyaluronique ou un sel de celui-ci, tel que l'hyaluronane sodique (également appelé généralement HA), ou un mélange de celles-ci. Le film multicouche polyélectrolyte est plus préférablement un film PLL/HA,.

Le film multicouches de polyélectrolites peut avantageusement être chargé avec une protéine tel qu'un facteur ostéoinductif. Un film chargé présente un intérêt particulier, car il agit comme un réservoir biomimétique pour le stockage et la libération de la protéine. Par exemple, la délivrance contrôlée de facteur de croissance à partir d'une surface de biomatériau peut être obtenue dans des conditions très satisfaisantes, car elle permet de concentrer commodément le facteur de croissance et de le libérer localement, et de le protéger de la dégradation des enzymes dans les fluides tissulaires, en particulier les protéases.

L'incorporation des proteines peut avoir lieu par adsorption ou diffusion ou par couplage desdits matériaux à au moins l'un des polyélectrolytes et adsorption dudit polyélectrolyte.

Dans un aspect particulier de l'invention, le polymère cationique comprenant un groupe amino est la poly (L-lysine).

Dans un aspect particulier de l'invention, le polymère anionique comprenant un groupement amino est l'acide hyaluronique.

Le film forme un revêtement sur l'échafaudage. Préférentiellement, le film revêt le maillage. Le maillage est enduit du film. Le film va ainsi recouvrir le maillage, plus précisément, les entretoises du maillage sont recouvertes. À titre préféré, la totalité du maillage est recouverte et donc chaque entretoise est recouverte. Le maillage est dit pelliculé. Le film recouvre de manière homogène le maillage.

Le film présente avantageusement une action ostéoinductrice. La présence du film de manière homogène sur le maillage assure une repousse osseuse homogène.

Le film comprend avantageusement des facteurs ostéoinducteurs et préférentiellement des protéines de la famille des protéines osseuses morphogénétique (BMPs). À ce titre le film joue un rôle biomimétique en délivrant des protéines BMP et en favorisant la repousse osseuse. Le biomimétisme étant l'application de connaissances issues de modèles biologiques.

À titre d'exemple préféré, les facteurs ostéoinducteurs sont des protéines de la famille des Bone Morphogenetic Proteins (BMPs), plus préférentiellement, BMP-2, BMP-7, BMP-4, BMP-9, BMP-6 ou un mélange de BMPs ou un hétéro-dimère de BMPs ou des fragments de BMPs ou des peptides dérivés de ces BMPs.

La suite de la description est faite en référence aux BMPs mais s'applique également aux autres facteurs ostéoinducteurs.

Le film forme un réservoir pour BMP correspondant avantageusement à un réservoir biomimétique. Les protéines de BMP sont avantageusement adsorbées au sein du film de façon non covalente ce qui permet un relargage in vivo après implantation du dispositif.

La quantité de BMPs dans le film est avantageusement sélectionnée de sorte que la quantité de BMPs au sein du dispositif soit entre 0,017mg/cm³ et 0,072mg/cm³, plus précisément entre 0,02 mg/cm³ et 0,08 mg/cm³. À titre d'exemple, la quantité de BMPs au sein d'un défaut osseux de 12 cm³ soit entre 240µg et 1000µg. La masse de BMPs est donnée par unité de volume de l'échafaudage. La quantité de BMP est ainsi réduite de 1/20 à 1/75 comparé aux éponges de collagènes commerciales.

Avantageusement, le dispositif selon l'invention permet une délivrance spatiale contrôlée des protéines BMPs. La délivrance des protéines BMPs est parfaitement contrôlée via l'architecture 3D, c'est à dire le maillage de l'échafaudage recouvert par le film. Cette délivrance spatiale contrôlée assure une repousse de l'os spatialement localisée au sein même du volume de l'échafaudage. La repousse osseuse dans le défaut osseux à réparer est de manière très avantageuse homogène.

Avantageusement, cette sélection de la quantité de BMPs assure l'absence d'inflammation locale, de gonflement, de pseudo-kyste osseux ou de résorption osseuse. De même, la quantité de BMP permet de limiter l'ossification à l'extérieur de l'implant.

Le film est avantageusement chargé en protéines BMP par trempage de l'échafaudage dans une solution présentant une concentration définie de protéines BMP.

Selon une possibilité, la quantité de protéines BMPs dans le film peut être modulée en fonction de la concentration des BMPs dans la solution dans lequel l'intégralité de l'échafaudage est trempée.

Selon une possibilité, la quantité de protéines BMPs dans le film peut être modulée en fonction les propriétés physico-chimiques du film, notamment son épaisseur et son degré de réticulation.

Selon un mode de réalisation, le film est un film polyélectrolyte multicouche. Avantageusement, le film est formé par une alternance de couches de poly(L-Lysine) (PLL) et d'acide hyaluronique (HA). Le film est formé d'un empilement de paires de couches, chaque paire comprenant des polymères de poly(L-Lysine) (PLL) et d'acide hyaluronique (HA). Une paire est également dénommée bi-couches. À titre d'exemple, le nombre de bicouches est de 24.

Selon un mode de réalisation, le film est réticulé préférentiellement par un agent de réticulation tel que 1-Éthyl-3-(3-diméthylaminopropyl) carbodiimide hydrochloride (EDC), préférentiellement à une concentration comprise entre 30 mg/mL (EDC30) et 70mg/ml (EDC70). La réticulation assure la formation de liaisons covalentes au sein du film.

Selon un mode de réalisation, le film est réticulé avant le chargement en facteurs ostéoinducteurs.

Le dispositif médical implantable selon l'invention est avantageusement stérilisable selon les exigences en vigueur pour ce type de dispositif tout en conservant ses propriétés et sa structure. Préférentiellement, le dispositif médical implantable activé est stérilisé tout en conservant ses propriétés et sa structure. La stérilisation peut être par exemple réalisée par gamma-irradiation.

Selon un aspect, l'invention concerne un procédé de fabrication d'un dispositif médical implantable tel que décrit ci-dessus comprenant une étape d'architecturation de l'échafaudage. L'architecturation correspond à la fabrication de l'échafaudage. La fabrication de l'échafaudage se fait par impression en 3 dimensions. La fabrication par impression de polymère permet de réaliser un implant dont la géométrie (taille, forme, porosité) est contrôlée dans les 3 dimensions.

Selon un exemple, l'étape de fabrication comprend le dépôt de filaments de PLA, à titre d'exemple d'environ 400 µm de diamètre suivant un motif prédéfini tel que par exemple -45 ° / 45 ° avec une hauteur de 2 mm et une distance d'espacement de ± 2 mm.

Selon un exemple, le film est fabriqué selon l'exemple 1. Le film est fabriqué par une étape de dépôt de couches successif par exemple de PLL et d'HA sur l'échafaudage.

Préférentiellement, l'étape de fabrication du film comprend une étape de réticulation et une étape de chargement du film en facteurs ostéoinducteurs. Selon un exemple, l'étape de réticulation est réalisée avant l'étape de chargement du film en facteurs ostéoinducteurs.

Selon une possibilité, le procédé de fabrication comprend une étape de stérilisation du dispositif médical implantable obtenu.

### Exemples

### Exemple 1 : Préparation d'échafaudages en PLA, revêtement de film multicouche de polyélectrolyte (PEM)

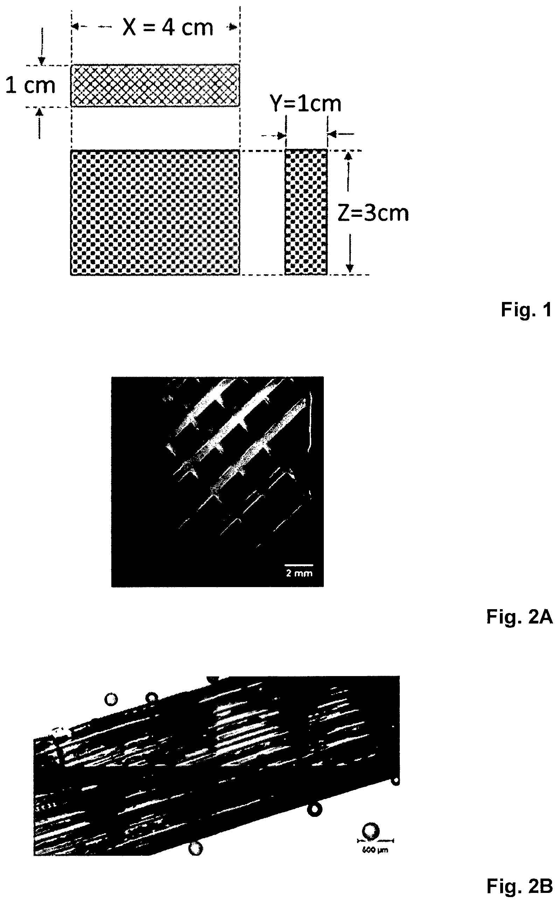

Des échafaudages, avantageusement biodégradables et biorésorbables, 10 x 30 x 40 mm parallélépipédiques en acide poly (lactique) de qualité médicale (Poly-Med, Inc, Lactoprene^{®} 100M Monofilament 1,75 mm) fabriqués par dépôt de fil fondu (3DXP - One) ont été réalisés. La figure 1 illustre un dispositif selon l'invention. Des filaments de PLA d'environ 400 µm de diamètre ont été déposés suivant un motif de -45° / 45° avec une hauteur de 2 mm et une distance d'espacement de ± 2 mm. L'échafaudage a une porosité de 85 % avec des pores entièrement interconnectés. Après la fabrication et avant le revêtement avec des films multicouches de polyélectrolytes, les échafaudages ont été stockés à l'abri de l'humidité dans un dessiccateur avec un gel de silice.

Les films multicouches de polyélectrolytes ont été déposés en utilisant 0,5 mg/mL de poly (L-lysine) (PLL, Sigma, France) et 1 mg / mL d'acide hyaluronique (HA, Lifecore, USA) et le robot de revêtement par immersion DR3 (Kirstein et Riegler GmbH) après dépôt à la main de 5 mg / mL de polyéthylèneimine (Aldrich). L'étendue de réticulation du film a été contrôlée en incubant les échafaudages revêtus dans 30 ou 70 mg/ml de 1-éthyl-3-(3-diméthylaminopropyl) carbodiimide (EDC, Sigma, France). Après stérilisation aux UV des implants, du BMP-2 (InductOs, Medtronic) a été post-chargé dans les films multicouches de polyélectrolytes à des concentrations de BMP initiales, dans la solution de chargement, de 20, 50 ou 110 µg/mL comme décrit précédemment dans les publications Bouyer M, Guillot R, Lavaud J, Plettinx C, Olivier C, Curry V, et al. Surface delivery of tunable doses of BMP-2 from an adaptable polymeric scaffold induces volumetric bone regeneration. Biomaterials. 104 (2016) 168-81 et Crouzier T, Ren K, Nicolas C, Roy C, Picart C. Layer-by-Layer films as a biomimetic reservoir for rhBMP-2 delivery: controlled differentiation of myoblasts to osteoblasts. Small. 5 (2009) 598-608. Enfin, les échafaudages enduits ostéoinducteurs ont été rincés, séchés et stockés à l'abri de l'humidité dans un dessiccateur avec un gel de silice jusqu'à l'implantation.

### Exemple 2 : Caractérisation des films multicouches de polyélectrolytes et quantification de la charge BMP-2.

La microscopie à fluorescence et la microscopie électronique à balayage ont été utilisées pour la caractérisation du revêtement du film sur l'échafaudage. Les films multicouches de polyélectrolytes séchés à l'air et enduits sur les échafaudages PLA ont été imagés par microscopie électronique à balayage (MEB) en utilisant un FEI-Quanta 250 SEM-FEG sous vide poussé à 15 keV en utilisant le détecteur Everhart-Thornley selon les publications Bouyer M, Guillot R, Lavaud J, Plettinx C, Olivier C, Curry V, et al. Surface delivery of tunable doses of BMP-2 from an adaptable polymeric scaffold induces volumetric bone regeneration. Biomaterials. 104 (2016) 168-81 et Crouzier T, Ren K, Nicolas C, Roy C, Picart C. Layer-by-Layer films as a biomimetic reservoir for rhBMP-2 delivery: controlled differentiation of myoblasts to osteoblasts. Small. 5 (2009) 598-608. Pour les observations de fluorescence, les échafaudages pelliculés ont été marqués avec PLL-FITC selon la publication Crouzier T, Sailhan F, Becquart P, Guillot R, Logeart-Avramoglou D, Picart C. The performance of BMP-2 loaded TCP/HAP porous ceramics with a polyelectrolyte multilayer film coating. Biomaterials. 32 (2011) 7543-54. imagés en utilisant un système de fluorescence Leica Macrofluo (Z16 Apo) en utilisant un objectif X0.8 et un microscope confocal Zeiss LSM 700 avec un objectif X.

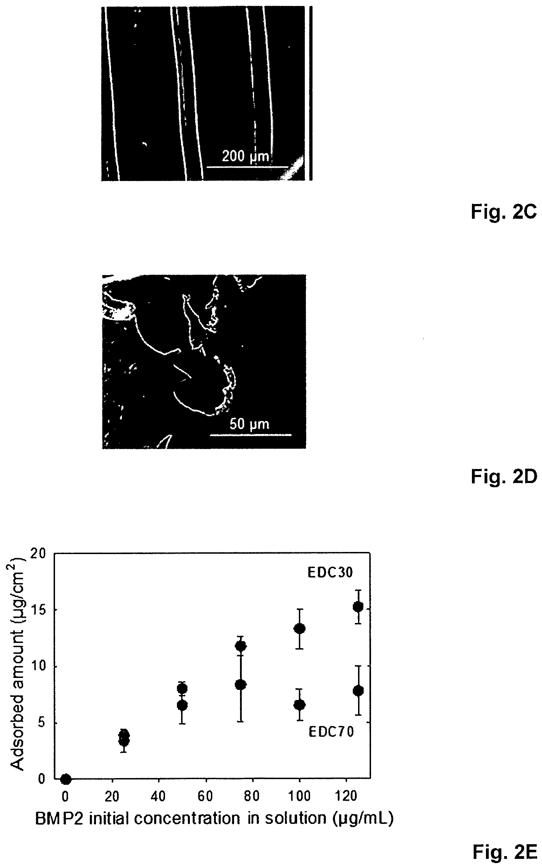

Les résultats obtenus sont visibles en figures 2A à 2D et permettent la visualisation d'un revêtement homogène de l'échafaudage. Le maillage de l'échafaudage est revêtu du film. La quantité de BMP-2 augmente avec la concentration initiale de BMP-2 dans la solution de chargement, mais elle atteint un plateau plus rapidement pour le film EDC70 (figure 2E). En ce qui concerne le BMP-2 sorti du film, il est plus élevé pour le film EDC30, le pourcentage maximum de BMP-2 libéré du film étant de l'ordre de 50% pour les films EDC30 par rapport à environ 20% pour les films EDC70 (Figure 2F).

La quantification de BMP-2 initialement chargée dans le film polyélectrolyte a été effectuée à l'aide d'un test de dosage d'acide micro bicinchoninine (microBCA) et le pourcentage de libération in vitro après plusieurs lavages avec un tampon physiologique (HEPES-NaCl), a été déterminé par spectrométrie de fluorescence en utilisant BMP-2 CF. Les résultats obtenus sont visibles aux figures 2E et 2F Pour la quantification du BMP-2 chargé dans les échafaudages pelliculés, un essai microBCA a été utilisé pour un faible BMP-2 (BMP20) tandis que Nanodrop (Thermofisher) a été utilisé pour des concentrations élevées. La concentration de BMP-2 dans la solution de chargement a été mesurée initialement puis après incubation avec l'échafaudage pelliculé. La quantité chargée correspond à la différence entre ces deux valeurs. Il est également exprimé en µg de protéines par volume d'échafaudage (µg/cm³).

**Tableau 1. Quantification de la quantité efficace de BMP-2 chargée dans l'échafaudage architectural revêtu d'un film. Le volume total de l'échafaudage était de 12 cm³ et sa surface efficace estimée à partir de la conception était de 144 cm2. Nous avons ciblé une charge de BMP-2 (masse unitaire par unité de volume d'échafaudage en µg/cm³). La quantité totale de BMP-2 chargée a été calculée pour chaque implant et rapportée en «masse par volume d'implant» (µg/cm³).**

| | Chargement Cible BMP-2 (µg/cm³) | BMP-2 totale chargée (µg/cm³) | Dose volumétrique chargée (µg/implant) |
|---|---|---|---|
| Petite dose (n = 2) | BMP20 | 240 µg | 20 |
| Dose élevée (n = 2) | BMP110 | 870 µg | 72,5 |
| Petite dose (n = 6) | BMP50 | 326 ± 80 µg | 27 |
| Dose élevée (n = 5) | BMP110 | 1000 ± 64 µg | 83 |

### Exemple 3 : Réparation d'un défaut mandibulaire de taille critique in vivo chez un mini-porc.

Pour optimiser la régénération osseuse, nous avons réalisé une expérience préliminaire sur les mandibules de mini-cochons. Nous avons initialement criblé dans 4 conditions différentes (n = 1), correspondant à deux niveaux de réticulation de film (EDC30 et EDC70) et deux concentrations de BMP-2 (BMP20 et BMP110). Ces concentrations exprimées en µg de BMP-2 par cm³ d'échafaudage sont les concentrations «volumiques» ciblées de BMP-2. Connaissant la surface efficace de l'échafaudage (144 cm²) et la quantité de BMP-2 chargée dans le film de polyélectrolyte, nous avons défini la concentration de BMP-2 dans la solution de chargement (en µg/mL) dans laquelle l'échafaudage a été trempé. Les quantités de BMP-2 effectivement chargées dans les échafaudages 3D pelliculés ont également été quantifiées (tableau 1). Deux contrôles négatifs ont été ajoutés : un défaut vide sans aucun implant et un défaut avec l'implant pelliculé, mais sans BMP-2.

Les animaux étaient en bonne santé. Il n'y avait pas d'infection postopératoire, d'échec d'implant ou de signe de trouble sanguin. Toutes les interventions chirurgicales se sont déroulées sans incident et il n'y a eu aucune complication chirurgicale. Pour un implant, il a fallu recouper le bord antérieur du défaut osseux afin d'améliorer l'ajustement avec l'implant. Toutes les plaques de titane étaient stables et fixées sur l'os natif (pas de desserrage ni de perte d'adhérence des vis). Lors de l'explantation, il était tout à fait impossible d'identifier macroscopiquement l'implant actif (chargé en BMP) de l'os natif à partir d'une reconstruction osseuse ou de tissus cicatriciels. La numération formule sanguine complète, l'haptoglobine et l'électrophorèse des protéines ont été mesurées pour évaluer l'inflammation et l'hémostase, l'ASAT, l'ALAT, la phosphatase alcaline (PAL), la gamma-glutamine transférase (GGT) et la bilirubinémie pour évaluer la fonction hépatique, la créatinine sérique et l'urée pour évaluer la fonction rénale. L'analyse des échantillons de sang n'a révélé aucune anomalie. Nous avons conclu que l'échafaudage avec ou sans le film et / ou le BMP-2 ne provoquait pas d'inflammation générale, ni de réaction hépatique ou rénale particulière en raison de cette expérience quelle que soit la condition.

Des acquisitions scanner ont été acquises au cours de la période de suivi. Pour chaque acquisition, un score CT-scan a été donné en aveugle par trois cliniciens. Les résultats sont illustrés aux Figures 3A et 3B. Les témoins négatifs n'ont pas montré de formation osseuse tout au long de la période de suivi. Tous les groupes BMP ont présenté une régénération osseuse, quelles que soient l'étendue de réticulation du film et la concentration de charge BMP-2, tandis que les témoins négatifs n'ont pas montré de formation osseuse.

Le score CT-scan a été utilisé pour calculer une valeur de plateau (Bmax) et un temps caractéristique de plateau (to T) en ajustant une fonction exponentielle aux données expérimentales.

| **BMP (µg/cm3)** | ***Bₘₐₓ* (A.U.)** | **T (Days)** | **R²** |
|---|---|---|---|
| 20 | 1.4 ± 0.0 | 21 ± 2 | 0.999 |
| 110 | 4.9 ± 0.7 | 60 ± 14 | 0.999 |

Pour les films EDC30, le tableau 1 reprend les valeurs calculées, les scores ont augmenté régulièrement avant d'atteindre une valeur plateau Bmax et étaient plus élevés pour le BMP110 que pour le BMP20 (4,9 + 0,7 contre 1,4 + 0,0, respectivement). Le temps pour atteindre le plateau (τ) était environ 3 fois plus rapide pour la faible dose que pour la forte dose (21 + 2 contre 60 + 14 jours).

| **BMP (µg/cm3)** | ***Bₘₐₓ* (A.U.)** | **T (Days)** | **R²** |
|---|---|---|---|
| 20 | 3.3 ± 0.4 | 30 ± 12 | 0.995 |
| 110 | 4.2 ± 3.0 | 88 ± 111 | 0.971 |

En revanche, pour les films EDC70, le tableau 2 reprend les valeurs calculées, l'ajustement exponentiel aux données était de mauvaise qualité pour la concentration de BMP-2 la plus élevée et il n'y avait pas de tendance claire dépendante de la dose. Pour la faible dose, Bmax était à 3,3 + 0,4 et τ à 30 + 12 jours.

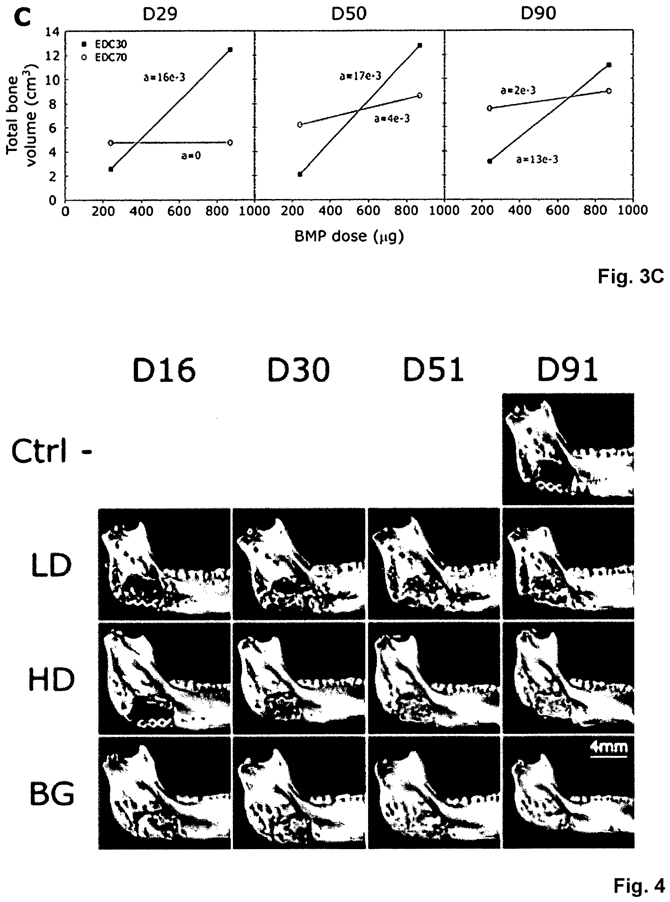

La quantification des volumes osseux régénérés totaux à partir des images CT pour les différents points dans le temps (D29, D50, D90) a confirmé la dose-dépendante BMP-2 de la réparation osseuse pour les films EDC30 et non pour les films EDC70. Les résultats sont illustrés à la figure 3C. La quantité d'os peu minéralisé et d'os hautement minéralisé a également été tracée. Les pentes des ajustements linéaires étaient plus élevées pour la partie fortement minéralisée, suggérant que ce type d'os a plus d'influence sur le volume osseux total que celui faiblement minéralisé.

Après 3 mois, les acquisitions µCT acquises après explantation des échafaudages ont été utilisées pour calculer le volume osseux total (BV) et la densité minérale osseuse (BMD) pour tous les échantillons. BV présentait également une nette dépendance à la dose de BMP-2, indépendamment du niveau de réticulation EDC, tandis que la DMO était plutôt stable. L'imagerie µCT des films EDC70 a confirmé qu'il n'y avait pas de dépendance à la dose de BMP-2 visible pour cette condition de film.

Dans l'ensemble, ces données ont établi la taille critique du défaut de l'os mandibulaire, la différence de cinétique de réparation osseuse en fonction de la dose de BMP-2 et l'influence du niveau de réticulation du film sur la quantité d'os nouvellement formé. Une nette dépendance à la dose de BMP-2 a été mise en évidence pour les films EDC30.

### Exemple 4 : L'influence de BMP-2 sur la cinétique de réparation osseuse et la quantité d'os hautement minéralisé.

Des expériences ont été répétées avec plus de mini-porcs par condition pour évaluer quantitativement l'effet des doses de BMP-2. Nous nous sommes concentrés uniquement sur les films EDC30 et avons sélectionné deux doses. Nous avons conservé la dose la plus élevée de BMP110 et augmenté la faible dose de BMP-2 à BMP50 (n = 6 dans chaque groupe). Nous avons ajouté une greffe osseuse comme contrôle positif (n = 4) et un échafaudage pelliculé comme contrôle négatif (film EDC30). De plus, un point de temps antérieur supplémentaire (D16) a été ajouté pour les acquisitions de tomodensitométriques.

Là encore, il n'y a pas eu de complication chirurgicale. Dans deux cas, la greffe osseuse était en deux pièces en raison de la petite taille de l'os iliaque (site donneur), mais dans tous les cas le défaut était complètement comblé. Dans trois cas (deux cas de greffe osseuse et un cas de forte dose de BMP-2), une petite collection séreuse a été trouvée autour de l'implant, et dans un cas une collection suppurée avec une fistule cutanée est apparue à J86.

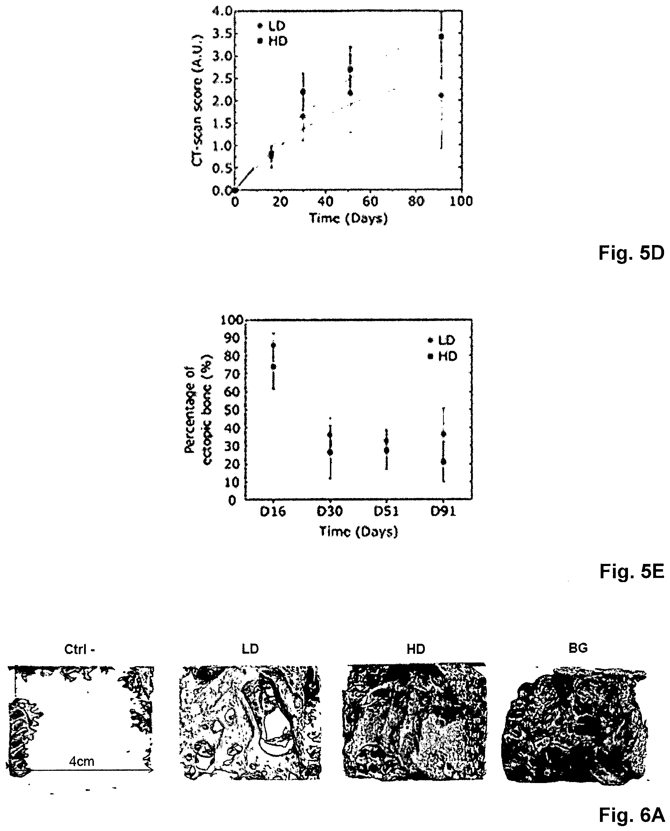

La figure 4 montre des représentations scanners en trois dimensions au fil du temps pour une autogreffe osseuse, la greffe osseuse synthétique obtenue pour les deux doses de BMP-2 et le témoin négatif. À première vue, nous avons observé que l'autogreffe osseuse se minéralisait avec le temps, mais que sa quantité totale ne changeait pas. En revanche, l'échafaudage pelliculé induit une réparation osseuse au fil du temps et la minéralisation était également visible. Le volume osseux total, les volumes osseux peu minéralisés et hautement minéralisés ont été quantifiés à partir des coupes tomodensitométriques (Figures 5, A, B, C). Le volume osseux total a augmenté au fil du temps pour atteindre un plateau, cette augmentation étant significativement plus élevée pour la condition de dose élevée de BMP-2 que pour la faible dose (figure 5A). La quantité de volume de greffe osseuse est restée constante. La quantité d'os peu minéralisé a rapidement augmenté avec le temps pour les BMP-2 faibles et élevés, mais sans différence statistique (figure 5B). En revanche, la quantité d'os hautement minéralisé a augmenté en fonction du temps, étant maximale à J51 (figure 5C). Il était significativement plus élevé pour la dose élevée que pour la faible dose de BMP-2.

Les scores CT-scan ont été tracés en fonction du temps et des données équipées d'un ajustement exponentiel (figure 5D). Le score a augmenté pour les deux doses de BMP-2, mais là encore avec des caractéristiques différentes : le Bmax était plus faible pour l'échafaudage avec la faible dose de BMP-2 que celui de la dose élevée de BMP-2 (2,9 + 1,2 contre 5,2 + 2,5). T était environ deux fois plus faible pour la faible BMP-2 (71 + 40 jours) que pour la dose élevée de BMP-2 (142 + 96 jours)

Nous avons ensuite analysé la partie de l'os régénéré qui se formait à l'extérieur de l'implant (figure 5E), ce que nous appelons ici un «os ectopique». Initialement élevée, la fraction d'os poussant à l'extérieur de l'implant a rapidement atteint une valeur de plateau autour de 28 à 35 %, indépendamment de la dose de BMP-2. Nous avons noté que la dispersion des valeurs était légèrement plus élevée pour la dose élevée, et également légèrement supérieure au point final de D91.

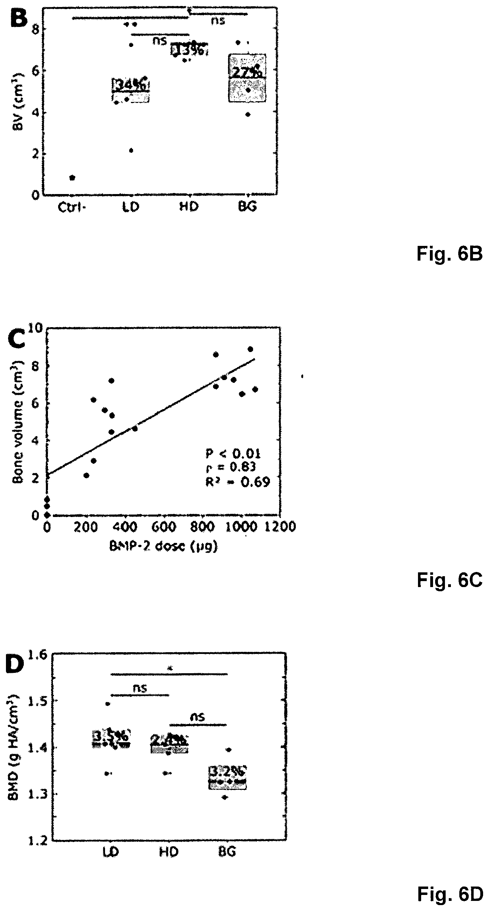

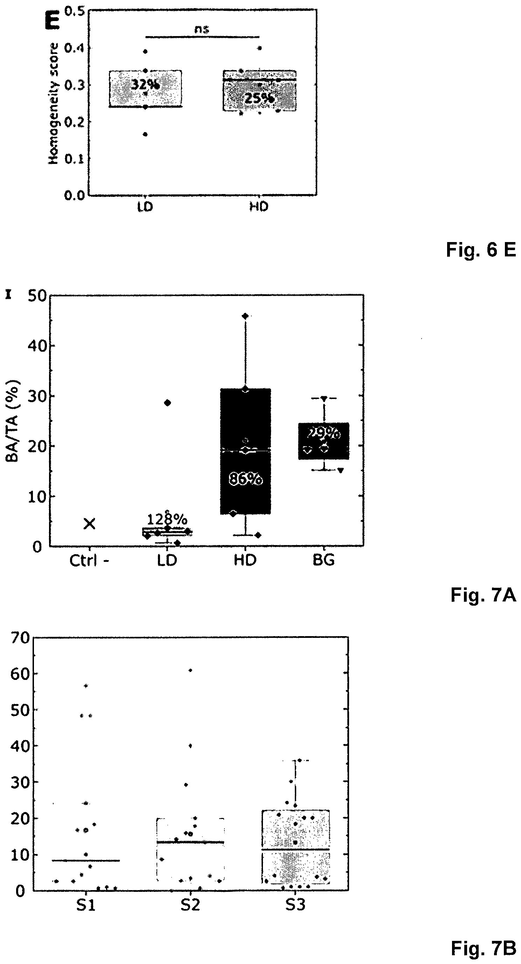

L'analyse µCT réalisée au jour 90 après le sacrifice du mini-porc a été utilisée pour analyser plus en détail l'os nouvellement formé. Les images µCT non présentées ici ont été réalisées dans les différentes conditions pour les échafaudages contenant des doses croissantes de BMP-2, prélevées dans les groupes expérimentaux. La dose de BMP-2 est donnée ici en masse totale par échafaudage. Le contrôle négatif confirme la taille critique du défaut osseux mandibulaire et les greffes osseuses fournissent une valeur de référence positive. Pour la faible dose de BMP-2, la formation d'os était rare. La quantité d'os augmentait progressivement et l'os nouvellement formé remplissait entièrement les pores de l'échafaudage de manière homogène. Il n'y avait aucun signe de formation osseuse ectopique excessive, même aux doses les plus élevées. Cela était également visible sur les images µCT reconstruites en 3D (Figure 7A). Le volume osseux était significativement plus élevé pour la dose élevée (valeur moyenne de 7,3 cm³) que pour la dose faible de BMP-2 (valeur moyenne de 4,9 cm³) (figure 6B) et était également plus élevé que pour la greffe osseuse de référence. De plus, la régénération osseuse était plus dispersée avec une faible dose, avec un coefficient de variation de 80,9% contre 20,1% pour la dose élevée (coefficient de variation de 20,1%). Lors du traçage de tous les volumes osseux expérimentaux en fonction de la dose de BMP-2 par implant, une corrélation linéaire a été trouvée (figure 6C). La DMO n'était pas significativement différente pour les différentes conditions (figure 6D) et elle était très homogène avec moins de 3,5% de variation pour chaque condition expérimentale. De plus, la quantité d'os développée à l'extérieur de l'implant ne dépendait pas de la dose de BMP-2. Enfin, le score d'homogénéité de l'os à l'intérieur de l'échafaudage était similaire pour les doses faibles et élevées de BMP-2 (Figure 6E).

Dans l'ensemble, ces données montrent que la formation osseuse à l'intérieur de l'échafaudage architecturé 3D est homogène, qu'il existe une formation osseuse dose-dépendante significative de BMP-2. La BMP-2 influence principalement la formation d'os minéralisé et n'induit pas la formation d'os ectopique.

### Exemple 5 : Score d'homogénéité osseuse.

L'implant de dimensions définies (3 cm x 4 cm x 1 cm, volume total TV de 12 cm³) a été pris comme région d'intérêt (ROI). Il a été séparé en dix tranches d'épaisseur égale le long de chaque axe (X, Y, Z). Pour chaque tranche, le rapport de volume osseux (BVr = BV / TV) a été calculé comme le volume osseux en une tranche (BVs) divisé par le volume de la tranche d'intérêt (correspondant à TV/10). Cette quantification a été faite pour chaque axe (Figure 7C) : l'écart type (SD) de BVr a été calculé et le score d'homogénéité a été défini comme la somme des trois SD sur les axes X, Y et Z.

Un examen histologique a révélé la présence d'os mature avec une structure haversienne caractéristique dans les implants chargés de BMP-2. L'interface entre l'os hôte (HB) et l'os nouvellement formé (NB) était visible. L'imagerie sous lumière non polarisée et polarisée a permis une meilleure visualisation des canaux haversiens et des connexions entre les ostéocytes. En outre, l'interface entre l'os hôte et l'os nouvellement formé était visible, car l'os hôte avait une structure plus lamellaire que l'os nouvellement formé. Certains ponts étaient visibles entre les deux types d'os, ce qui peut contribuer à augmenter la résistance mécanique de l'os nouvellement formé. Dans certains cas, en particulier pour la HD (haute dose), la différence entre l'os natif et l'os nouveau n'était même pas distinguable. Dans le cas de l'autogreffe osseuse (BG), l'os hôte et l'os greffé étaient en contact direct ou séparés par du tissu mésenchymateux. En l'absence de BMP-2, seul le tissu mésenchymateux (m) s'est formé. Avec une faible dose LD de BMP-2, la quantité de nouvel os était faible et le tissu mésenchymateux était visible. La surface osseuse sur la surface totale (BA / TA en%) a été quantifiée sur la base de ces images (figure 7A). En accord avec les quantifications CT et µCT, plus d'os a été formé pour le HD, dont la valeur médiane était similaire BG. Enfin, l'homogénéité de l'os nouvellement formé à l'intérieur de l'architecture 3D a été quantifiée (Figure 7B). Là encore, la formation osseuse était similaire pour les trois coupes de l'échantillon, prouvant l'homogénéité de la formation osseuse.

Dans la présente demande à titre d'exemple les analyses statistiques sont réalisées comme décrits ci-dessous.

OriginPro (OriginLab), Excel (Microsoft Office) et R pour mac OS X (Fondation R pour le calcul statistique, CRAN) ont été utilisés pour toutes les analyses. Les données ont été exprimées en moyenne ± écart type. Les données non paramétriques ont été présentées par intervalle médian et interquartile. Les différences entre les groupes ont été évaluées par analyse de variance (ANOVA) et analyse post-hoc de Bonferroni ou test t de Student. Les différences entre les groupes à des valeurs p <0,05 (*) et p <0,01 (**) ont été considérées comme significatives.

L'invention n'est pas limitée aux modes de réalisations précédemment décrits et s'étend à tous les modes de réalisation couverts par les revendications.

## Revendications

1. Dispositif médical implantable pour la réparation osseuse d'une perte de substance osseuse comprenant :
- un échafaudage présentant une structure tridimensionnelle et comprenant au moins un polymère,
- un film comprenant au moins une protéine de la famille des Bone Morphogenetic Proteins (BMPs),
**caractérisé en ce que** l'échafaudage définit un volume intérieur comprenant un maillage tridimensionnel délimitant des pores, les pores étant ouverts et interconnectés, chaque pore ayant une plus grande dimension supérieure à 200µm, l'échafaudage présentant une porosité minimale de 80%,
et **en ce que** le film revêt le maillage tridimensionnel.

2. Dispositif selon la revendication précédente dans lequel l'échafaudage est inerte.

3. Dispositif selon l'une quelconque des revendications précédentes dans lequel l'échafaudage comprend au moins un polymère choisi parmi un groupe constitué d'acide polylactique (PLA), d'acide polyglycolique (PGA), de polycaprolactone (PCL), ou des copolymères ou des mélanges de ceux-ci.

4. Dispositif selon la revendication précédente dans lequel le maillage tridimensionnel présente des angles d'orientation compris entre 0° et 120°.

5. Dispositif selon l'une quelconque des revendications précédentes comprenant une quantité de protéine BMPs comprise entre 0,017mg/cm³ à 0,072mg/cm³.

6. Dispositif selon l'une quelconque des revendications précédentes dans lequel le film est un film comprenant des polyélectrolytes.

7. Dispositif selon l'une quelconque des revendications précédentes dans lequel le film est un film polyélectrolyte multicouche réticulé.

8. Dispositif selon l'une quelconque des revendications précédentes dans lequel le film réticulé à une concentration comprise entre 30 mg/mL et 70mg/mL d'1-Éthyl-3-(3-diméthylaminopropyl)carbodiimide hydrochloride (EDC).

9. Dispositif selon l'une quelconque des revendications précédentes pour la réparation osseuse de volume compris entre 2 cm³ et 15 cm³.

10. Procédé de fabrication d'un dispositif médical implantable selon l'une quelconque des revendications précédentes **caractérisé en ce que** l'échafaudage est fabriqué par impression 3D.

11. Procédé de fabrication selon la revendication précédente comprenant une étape de stérilisation du dispositif médical implantable selon l'une quelconque des revendications 1 à 9.
